# EUROPÄISCHE PATENTSCHRIFT

(11) **EP 0 501 369 B1**
(45) Veröffentlichungstag und Bekanntmachung des Hinweises auf die Patenterteilung: **28.12.1994**
(21) Anmeldenummer: 92103050.8
(22) Anmeldetag: 24.02.1992
(51) Int. Cl.: C07C 311/65

(54) **Verfahren zur Herstellung von Sulfonylisocyanaten**
Process for the preparation of sulphonyl isocyanates
Procédé pour la préparation de sulfonylisocyanates

(30) Priorität: 25.02.1991 DE 4105823
(43) Veröffentlichungstag der Anmeldung: 02.09.1992
(73) Patentinhaber: Hoechst Schering AgrEvo GmbH, 13342 Berlin (DE)
(72) Erfinder: Schlegel, Günther, Dr., W-6237 Liederbach (DE); Lachhein, Stephen, Dr., W-6238 Hofheim am Taunus (DE); Berger, Harald, Dr., W-6233 Kelkheim (Taunus) (DE)

(56) Entgegenhaltungen:
- EP-A- 0 053 331
- EP-A- 0 131 258
- EP-A- 0 342 569

## Beschreibung

Die Erfindung betrifft das Gebiet der technischen Verfahren zur Herstellung reaktiver Verbindungen, die zum Beispiel zur Herstellung von Pflanzenschutzmitteln, wie Sulfonylharnstoffen, verwendet werden können.

Gegenstand der vorliegenden Erfindung ist ein Verfahren zur Herstellung eines Sulfonylisocyanats der Formel I

R¹ - X - SO₂ - N = C = O (I)

worin
- X: O oder NR²
- R¹: (C₁-C₄)-Alkylsulfonyl oder Phenyl, das unsubstituiert oder ein- oder mehrfach durch Reste aus der Gruppe Halogen, (C₁-C₄)-Alkyl und (C₁-C₄)-Alkoxy substituiert ist, und
- R²: (C₁-C₄)-Alkyl
bedeuten,
durch Umsetzung einer Verbindung der Formel II,

R¹ - X - H (II)

worin
X und R¹ die in Formel I genannten Bedeutungen haben, mit dem Chlorsulfonylisocyanat der Formel III

Cl - SO₂ - N = C = O (III)

zu einer Verbindung der Formel IV,

R¹ - X - CO - NH - SO₂ - Cl (IV)

worin R¹ und X die in Formel I genannten Bedeutungen haben, und deren anschließende Umlagerung zu der Verbindung der Formel I, dadurch gekennzeichnet, daß man das Verfahren ohne Lösungsmittel oder in Gegenwart eines inerten organischen Lösungsmittels kontinuierlich durchführt.

Bevorzugt sind die Verbindungen der Formel I, worin R¹ = CH₃SO₂, X = NR² und R² = CH₃ sind oder R¹ = 2-Ethoxyphenyl und X = O bedeuten.

Die Verbindungen der Formel I sind bekannt. Sie finden Verwendung als wertvolle Zwischenprodukte bei der Herstellung von Sulfonylharnstoffen mit herbizider Wirkung (EP-A-071 958; EP-A-342,569; EP-A-0 131 258).

Die Verbindungen der Formel II sind entweder kommerziell erhältlich oder lassen sich nach literaturbekannten Methoden leicht darstellen (DE-A-1 929 295).

Bekannt ist, daß man die Verbindungen der Formel I nach ein- bzw. zweistufigen Umsetzungen diskontinuierlich in 60 bis 80 % d.Th. Ausbeute herstellen kann (DE-A-22 57 240; G. Lohaus, Chem. Ber. 105, 2791-2799 (1972)).

Es ist auch ein diskontinuierliches Verfahren zur Herstellung von 2-Ethoxyphenoxysulfonylisocyanat aus Chlorsulfonylisocyanat und 2-Ethoxyphenol in Xylol bekannt (EP-A-0 342 569). Trotz in der Regel hoher Rohausbeute ist die isolierte Ausbeute an Ethoxyphenoxysulfonylisocyanat hierbei lediglich 91 % d.Th. (siehe Vergleichsbeispiele I und II).

Diese bekannten diskontinuierlichen Verfahren lassen somit hinsichtlich der Ausbeute an Sulfonylisocyanaten zu wünschen übrig und ergeben Rückstände an Ausgangsstoffen oder Nebenprodukten, die entsorgt werden müssen. Dies führt im technischen Maßstab zu großen Belastungen sowohl aus ökonomischer als auch ökologischer Sicht.

Bei den bekannten diskontinuierlichen Verfahren ist eine minimale Reaktionszeit von etwa 2,5 Stunden nötig;, die Umwandlung der Verbindungen der Formel IV zu den Verbindungen der Formel I ist somit sehr langsam. Die Geschwindigkeit der Umlagerungsreaktion kann durch Temperaturerhöhung nicht beliebig gesteigert werden, weil die Sulfonylisocyanate bei höheren Temperaturen thermisch labil sind.

Alle oben geschilderten Nachteile vermeidet das erfindungsgemäße Verfahren, bei dem die Sulfonylisocyanate der Formel I in nahezu quantitativen Ausbeuten von etwa 98 % d.Th. oder darüber und in so hohen Reinheiten erhalten werden, daß diese Produkte ohne zusätzliche Reinigungsoperationen direkt in Folgeumsetzungen eingesetzt werden können. Zudem ist das Verfahren aufgrund seiner kontinuierlichen Fahrweise verfahrenstechnisch besonders einfach zu handhaben.

Das erfindungsgemäße Verfahren wird z.B. so durchgeführt, daß man die Verbindungen der Formel II und III in einem Vorreaktor kontinuierlich zu den Verbindungen der Formel IV umsetzt und diese dann durch ein Strömungsrohr oder eine Kaskadenanordnung von geeigneter Länge kontinuierlich durchpumpt und reagieren läßt. Die entstehende gasförmige HCl wird vorzugsweise am Ende des Strömungsrohres über Kopf abdestilliert. Bei der Kaskadenanordnung ist es auch möglich, HCl schon bei einigen oder allen Kaskadenstufen abzudestillieren.

Für 1 Mol der Verbindung der Formel II werden vorzugsweise 1 - 1,2 Moläquivalente der Verbindung der Formel III eingesetzt.

Das Verfahren kann ohne Lösungsmittel oder in einem geeigneten, unter den Reaktionsbedingungen vor allem gegen Chlorsulfonylisocyanat inerten Lösungsmittel, wie halogenierte oder nitrierte aromatische Kohlenwasserstoffe, z.B. Chlorbenzol und Dichlorbenzol bzw. Nitrobenzol durchgeführt werden.

Die Reaktionstemperatur für die Umlagerung der Verbindung der Formel IV liegt im Strömungsrohr oder der Kaskadenanordnung vorzugsweise zwischen 100° und 150°C.

Die Reaktionstemperatur zur Darstellung der Verbindung der Formel IV liegt vorzugsweise zwischen 20 und 100°C, kann jedoch insbesondere auch höher sein, z.B. bis zu 150°C. Im Falle der Verwendung eines Vorreaktors läßt sich diese Temperatur gut einstellen.

Es kann aber auch so verfahren werden, daß die Verbindungen der Formeln II und III zusammen über 2 getrennte Pumpen simultan in ein Strömungsrohr eingepumpt werden und im Strömungsrohr zunächst z.B. an einem statischen Mischer vermischt werden. Im Strömungsrohr laufen dann sowohl die Reaktion der Verbindungen der Formeln II und III als auch die Umlagerung zu Verbindungen der Formel I ab. Hierbei kann es von Vorteil sein, wenn man in dem Strömungsrohr einen Temperaturgradienten einstellt oder zwei oder mehrere Rohre mit unterschiedlicher Manteltemperatur hintereinander durchfährt.
Die Rohre können mit Füllkörpern bestückt sowie ganz oder teilweise mit der Reaktionsmischung gefüllt sein.

In einer weiteren Ausgestaltung des Verfahrens werden die Verbindungen der Formeln II und III simultan, oder nach Vorreaktion zur Verbindung der Formel IV, die Verbindung der Formel IV von oben in eine vertikale Reaktionskolonne (z.B. Destillationskolonne mit Füllkörpern) dosiert und zugleich von unten organisches Lösungsmittel entgegengeführt und über Kopf zusammen mit der HCl abdestilliert.

In bevorzugter Ausgestaltung wird das kontinuierliche Verfahren so durchgeführt, daß man in einem Vorreaktor die Verbindung der Formel IV darstellt und die erhaltene Reaktionsmischung durch eine Kaskadenanordnung aus mehreren verhältnismäßig kleinen Reaktionsgefäßen kontinuierlich strömen läßt und entstehende HCl auf jeder Kaskadenstufe abdestilliert. Auch bei diesem Verfahren wird vorzugsweise ein Temperaturgradient eingestellt.

Bevorzugt ist auch eine Kombination aus mehreren der genannten Verfahrensausgestaltungen, wobei man nach der kontinuierlichen Bildung der Verbindungen IV im Vorreaktor die Reaktionsmischung zunächst in ein kleineres Reaktionsgefäß kontinuierlich und anschließend durch ein Strömungsrohr geeigneter Länge strömen läßt.

Die Reaktorverweilzeit für die Umlagerung der Verbindung der Formel IV ist vorzugsweise bei 30 bis 90 min, insbesondere 45 bis 75 min. Sie hängt von der Dimension der Reaktoren und den Durchflußmengen der Reaktionspartner ab.

Die Reaktorverweilzeit für die schnelle Vorreaktion kann sehr kurz gewählt werden, z.B. 1 bis 10 min.

Die Isolierung der hergestellten Sulfonylisocyanate kann nach üblichen Methoden erfolgen, beispielsweise durch Destillation unter reduziertem Druck.

Das erfindungsgemäße Verfahren ermöglicht die Herstellung von Sulfonylisocyanaten der Formel (I) in technischem Maßstab in überraschend hoher Ausbeute unter leicht zu kontrollierenden Bedingungen. Das Verfahren ist besonders überraschend im Hinblick auf die verhältnismäßig langsame Umlagerung der Verbindung der Formel IV, für die im Batch-Verfahren Verweilzeiten im Stundenbereich erforderlich sind. Je langsamer eine Reaktion abläuft, umso geringer sind üblicherweise die Vorteile, die beim Übergang vom Batch-Verfahren zum kontinuierlichen Verfahren zu erreichen sind. Das erfindungsgemäße Verfahren ermöglicht jedoch eine unerwartet bessere Ausbeute unter einfach zu kontrollierenden Reaktionsbedingungen.

### Beispiel 1

### Methansulfonyl-methylamino-sulfonylisocyanat

In einem 20 l-Vierhalskolben werden 5 l trockenes Chlorbenzol bei 60°C vorgelegt und kontinuierlich innerhalb von 15 Stunden jeweils mit einer separaten Pumpe 15,1 kg Methansulfonsäure-methylamid gelöst in 20 kg Chlorbenzol, und 20,5 kg Chlorsulfonylisocyanat, gelöst in 15 kg Chlorbenzol, zudosiert. Während dieser Zugabe werden über ein Steigrohr ca. 5 kg/Stunde der Reaktionslösung aus dem Reaktionsgefäß gepumpt und in ein senkrecht stehendes Strömungsrohr von 4 m Länge und 4 cm Durchmesser gepumpt. Die Manteltemperatur des Strömungsrohres beträgt 140 - 150°C. Über Kopf wird die entstehende Salzsäure abdestilliert und die Reaktionslösung kontinuierlich ablaufen gelassen.

Nach Spülen des Vierhalskolbens und des Strömungsrohres mit 20 kg Chlorbenzol und Abdestillieren des Lösungsmittels Chlorbenzol vom Strömungsfiltrat erhält man 29,6 kg Methansulfonyl-methylamino-suffonylisocyanat mit einem Gehalt von 98,2 Gew.-%, was einer Ausbeute von 98,0 % d.Th. entspricht.

Der Siedepunkt einer repräsentativen Probe lag bei 95 - 97°C/0,13 mbar. Das ¹H-NMR-Spektrum des Rohproduktes entspricht hinsichtlich Identität und Reinheit einer destillierten Probe aus einem literaturbeschriebenen Verfahren.

### Beispiel 2

### 2-Ethoxyphenoxy-sulfonylisocyanat

In den ersten Kolben einer mit Chlorbenzol gefüllten Kaskadenanordnung, bestehend aus drei 1 l-Vierhalskolben mit Überlauf und Destillationsaufsatz, werden über 6 Stunden 6,9 kg 2-Ethoxyphenol und 7,2 kg Chlorsulfonylisocyanat gleichmäßig eindosiert und der Ablauf des dritten Kolbens in einem Vorratsbehälter gesammelt. Die Apparatur wird unter Stickstoffatmosphäre gehalten. Der erste Kolben wird in einem 90°C warmen Bad, der zweite in einem 150°C warmen Bad und der dritte in einem 165°C warmen Bad temperiert. Aus den Kolben wird während der unter starkem Rückfluß verlaufenden Reaktion die entstehende HCl über Kopf in eine geeignete Vorlage getrieben und geringe Mengen an abdestilliertem Chlorbenzol in den 1. Kolben zurückgeführt. Nach Beendigung des Durchsatzes wird die Apparatur mit 5 l Chlorbenzol gespült und in den Vorratsbehälter entleert. Abdestillieren des Lösungsmittels ergibt 12,3 kg Rückstand, der laut HPLC-Analyse (nach Derivatisierung) 96,2 Gew.-% 2-Ethoxyphenoxysulfonylisocyanat enthält, was einer Ausbeute von 97,3 % der Theorie entspricht. Eine bei 140° C/0,67 mbar destillierte Probe hat einen Brechungsindex von n_{D}²⁵ = 1,5045 und stimmt auch im ¹H-NMR-Spektrum mit nach bekannten Verfahren hergestelltem Material überein.

### Vergleichsbeispiel I

### (Darstellung von 2-Ethoxyphenoxysulfonylisocyanat in Xylol nach EP-A-342 569)

55,2 g 2-Ethoxyphenol werden in 200 ml Xylol gelöst und bei 25°C mit 67,9 g Chlorsulfonylisocyanat umgesetzt. Die Reaktionsmischung wird auf 140°C erwärmt und unter Rückflußbedingungen für 2,5 Stunden erhitzt, wobei HCl entsteht. Nach Abdestillieren des Lösungsmittels erhält man 97,2 g eines Öls, das 91%ige Reinheit bezogen auf das Gewicht besitzt, was einer Ausbeute von 91 % d.Th. entspricht.

### Vergleichsbeispiel II

### (Darstellung von 2-Ethoxyphenylisocyanat in Chlorbenzol unter EP 342 569)

55,2 g 2-Ethoxyphenol werden in 200 ml Chlorbenzol gelöst und bei 25°C mit 67,9 g Chlorsulfonylisocyanat umgesetzt. Die Reaktionsmischung wird auf 132°C erwärmt und unter Rückflußbedingungen für 2,5 Stunden erhitzt, wobei HCl entsteht. Nach Abdestillieren des Lösungsmittels erhält man 91,8 g eines Öls, das 94,8 Gew.-% des gewünschten Produkts enthält, was einer Ausbeute von 89,5 % d.Th. entspricht.

## Patentansprüche

1. Verfahren zur Herstellung einer Verbindung der Formel I,
R¹ - X - SO₂ - N = C = O (I)
worin
X O oder NR²
R¹ (C₁-C₄)-Alkylsulfonyl oder Phenyl, das unsubstituiert oder ein- oder mehrfach durch Reste aus der Gruppe Halogen,
(C₁-C₄)-Alkyl und (C₁-C₄)-Alkoxy substituiert ist, und
R² (C₁-C₄)-Alkyl
bedeuten, durch Umsetzung einer Verbindung der Formel II,
R¹ - X - H (II)
worin X und R¹ die in Formel I genannten Bedeutungen besitzen, mit dem Chlorsulfonylisocyanat der Formel III
Cl - SO₂ - N = C = O (III)
zu einer Verbindung der Formel IV,
R¹ - X - CO - NH - SO₂-Cl (IV)
worin R¹ und X die in Formel I genannten Bedeutungen besitzen, und deren anschließende Umlagerung zu der Verbindung der Formel I, dadurch gekennzeichnet, daß man das Verfahren ohne Lösungsmittel oder in Gegenwart eines inerten organischen Lösungsmittels kontinuierlich durchführt.

2. Verfahren nach Anspruch 1, dadurch gekennzeichnet, daß die Reaktionstemperatur für die Umsetzung der Verbindungen der Formeln II und III zwischen 20 und 150°C ist.

3. Verfahren nach Anspruch 2, dadurch gekennzeichnet, daß die Temperatur zwischen 20 und 100°C ist.

4. Verfahren nach einem oder mehreren der Ansprüche 1 bis 3, dadurch gekennzeichnet, daß die Reaktionstemperatur für die Umlagerung der Verbindung der Formel IV 100 bis 150°C beträgt.

5. Verfahren nach einem oder mehreren der Ansprüche 1 bis 4, dadurch gekennzeichnet, daß als Lösungsmittel ein halogenierter oder nitrierter aromatischer Kohlenwasserstoff eingesetzt wird.

6. Verfahren nach Anspruch 5, dadurch gekennzeichnet, daß Chlorbenzol als Lösungsmittel eingesetzt wird.

7. Verfahren nach einem oder mehreren der Ansprüche 1 bis 6, dadurch gekennzeichnet, daß das Verfahren mit oder ohne Vorreaktor in einem Strömungsrohr, einer Kaskadenanordnung oder einer Reaktionskolonne mit Füllkörpern oder einer Kombination daraus durchgeführt wird.

8. Verfahren nach Anspruch 7, dadurch gekennzeichnet, daß das ganze Verfahren in einem Strömungsrohr durchgeführt wird.

9. Verfahren nach Anspruch 7, dadurch gekennzeichnet, daß bei dem Verfahren ein Vorreaktor für die Umsetzung der Verbindungen der Formeln II und III zur Verbindung der Formel IV und eine Kaskadenfolge mit mehreren Reaktionsgefäßen für die Umlagerung der Verbindung der Formel IV eingesetzt wird, wobei jedes folgende Reaktionsgefäß höhere Temperatur als das vorangehende aufweist.

10. Verfahren nach einem oder mehreren der Ansprüche 1 bis 8 oder 1 bis 7 und 9, dadurch gekennzeichnet, daß die Reaktorverweilzeit für die Umlagerungsreaktion 30 bis 90 min beträgt.

11. Verfahren nach Anspruch 10, dadurch gekennzeichnet, daß die Reaktorverweilzeit 45 bis 75 min beträgt.

12. Verfahren nach einem oder mehreren der Ansprüche 1 bis 8,10 und 11 oder 1 bis 7 und 9 bis 11, dadurch gekennzeichnet, daß man auf 1 Mol Verbindung der Formel II bis 1,2 Mol Verbindung der Formel III einsetzt.

13. Verfahren nach einem oder mehreren der Ansprüche 1 bis 8,10 bis 12 oder 1 bis 7, 9 bis 12, dadurch gekennzeichnet, daß R¹ = CH₃SO₂, X = NR² und R² = CH₃ sind.

14. Verfahren nach einem oder mehreren der Ansprüche 1 bis 8, 10 bis 12 oder 1 bis 7, 9 bis 12, dadurch gekennzeichnet, daß R¹ = 2-Ethoxyphenyl und X = O ist.

## Claims

1. A process for the preparation of a compound of the formula I
R¹ - X - SO₂ - N = C = O (I)
in which
X is O or NR²,
R¹ is (C₁-C₄)alkylsulfonyl or phenyl which is unsubstituted or monosubstituted or polysubstituted by radicals selected from the group comprising halogen, (C₁-C₄)alkyl and (C₁-C₄)-alkoxy, and
R² is (C₁-C₄)alkyl,
by reacting a compound of the formula II
R¹ - X - H (II)
in which X and R¹ have the meanings given in formula I, with the chlorosulfonyl isocyanate of the formula III
Cl - SO₂ - N = C = O (III)
to give a compound of the formula IV
R¹ - X - CO - NH - SO₂ - Cl (IV)
in which R¹ and X have the meanings given in formula I, and the subsequent rearrangement of the compound of the formula IV to give the compound of the formula I, which comprises carrying out the process continuously without a solvent or in the presence of an inert organic solvent.

2. The process as claimed in claim 1, wherein the reaction temperature for the reaction of the compounds of the formulae II and III is between 20 and 150°C.

3. The process as claimed in claim 2, wherein the temperature is between 20 and 100°C.

4. The process as claimed in one or more of claims 1 to 3, wherein the reaction temperature for the rearrangement reaction of the compound of the formula IV is 100 to 150°C.

5. The process as claimed in one or more of claims 1 to 4, wherein the solvent employed is a halogenated or nitrogenated aromatic hydrocarbon.

6. The process as claimed in claim 5, wherein chlorobenzene is used as the solvent.

7. The process as claimed in one or more of claims 1 to 6, wherein the process is carried out with or without a first reactor in a flow tube, a cascade arrangement or a packed reaction column, or a combination of these.

8. The process as claimed in claim 7, wherein the entire process is carried out in a flow tube.

9. The process as claimed in claim 7, wherein, in the process, a first reactor is used for reacting the compounds of the formulae II and III to give the compound of the formula IV and a sequence of cascades with a plurality of reaction vessels is used for the rearrangement reaction of the compound of the formula IV, where each subsequent reaction vessel has a higher temperature than the preceding one.

10. The process as claimed in one or more of claims 1 to 8 or 1 to 7 and 9, wherein the reactor residence time for the rearrangement reaction is 30 to 90 minutes.

11. The process as claimed in claim 10, wherein the reactor residence time is 45 to 75 minutes.

12. The process as claimed in one or more of claims 1 to 8, 10 and 11 or 1 to 7 and 9 to 11, wherein 1 to 1.2 moles of compound of the formula III are employed per mole of compound of the formula II.

13. The process as claimed in one or more of claims 1 to 8, 10 to 12 or 1 to 7, 9 to 12, wherein R¹ is CH₃SO₂, X is NR² and R² is CH₃.

14. The process as claimed in one or more of claims 1 to 8, 10 to 12 or 1 to 7, 9 to 12, wherein R¹ is 2-ethoxyphenyl and X is O.

## Revendications

1. Procédé de préparation d'un composé de formule I :
R¹ - X - SO₂ - N = C = O (I)
(dans laquelle
X représente O ou NR²;
R¹ représente un groupe alkyl sulfonyle en C₁ à C₄ ou phényle, qui peut être non substitué ou être substitué plusieurs fois par des restes choisis parmi par un atome d'halogène, un reste alkyle en C₁ à C₄ et alcoxy en C₁ à C₄, et
R² représente un groupe alkyle en C₁ à C₄),
par réaction d'un composé de formule II,
R¹- X - H (II)
(dans laquelle X et R¹ ont les sens indiqués pour la formule I) avec de l'isocyanate de chlorosulfonyle de formule III :
Cl - SO₂ - N = C = O (III)
pour obtenir un composé
R¹ - X - CO - NH - SO₂ - Cl (IV)
[dans laquelle R¹ et X ont les sens indiqués dans le cas de la formule I] et dont on effectue une transposition subséquente donnant le composé de formule I, procédé caractérisé en ce qu'on conduit le procédé en continu, sans solvant ou en présence d'un solvant organique inerte

2. Procédé selon la revendication 1, caractérisé en ce que la température pour la réaction des composés de formules II et III se situe entre 20 et 100°C.

3. Procédé selon une ou plusieurs des revendications 2 à 3, caractérisé en ce que la température pour la réaction de transposition se situe entre 20 et 100°C.

4. Procédé selon une ou plusieurs des revendications 1 à 3, caractérisé en ce que la température pour la réaction de transposition du composé de formule IV va de 100 à 150°C.

5. Procédé selon une ou plusieurs des revendications 1 à 4, caractérisé en ce qu'on utilise comme solvant un hydrocarbure aromatique, halogéné ou nitré.

6. Procédé selon la revendication 5, caractérisé en ce qu'on utilise du chlorobenzène comme solvant.

7. Procédé selon une ou plusieurs des revendications 1 à 6, caractérisé en ce qu'on conduit le procédé, avec ou sans réacteur préliminaire, dans un tube à écoulement, dans un agencement en cascade ou dans une colonne de réaction comportant des corps de garnissage ou dans une combinaison de ces moyens.

8. Procédé selon la revendication 7, caractérisé en ce que la totalité du procédé est mise en oeuvre dans un tube pour écoulement.

9. Procédé selon la revendication 7, caractérisé en ce que, dans le procédé on utilise un pré-réacteur pour la réaction des composés de formule II et III donnant le composé de formule IV et une suite en cascade comportant plusieurs réacteurs pour la réaction de transposition du composé de formule IV, chaque réacteur suivant ayant une température plus élevée que le réacteur qui le précède.

10. Procédé selon une ou plusieurs des revendications 1 à 8 ou 1 à 7 et 9, caractérisé en ce que le temps de séjour pour la réaction de transposition est de 30 à 90 minutes.

11. Procédé selon la revendication 10, caractérisé en ce que le temps de séjour pour la réaction est de 45 à 75 minutes.

12. Procédé selon une ou plusieurs des revendications 1 à 8, 10 et 11 ou bien 1 à 7 et 9 à 11, caractérisé en ce qu'on utilise pour 1 mole du composé de formule II, 1 à 1,2 mole du composé de formule III.

13. Procédé selon les revendications 1 à 8, 10 à 12 ou bien 1 à 7, 9 à 12, caractérisé en ce que R¹ représente CH₃SO₂, X représente NR² et R² représente CH₃.

14. Procédé selon une ou plusieurs des revendications 1 à 8, 10 à 12 ou bien 1 à 7, 9 à 12, caractérisé en ce que R¹ représente un groupe 2-éthoxyphényle et X représente O.
